# EUROPEAN PATENT APPLICATION

(11) **EP 4 595 880 A1**
(43) Date of publication of application: **06.08.2025**
(21) Application number: 23869612.4
(22) Date of filing: 05.05.2023
(51) Int. Cl.: A61B 5/145

(54) **ANALYTE SENSOR FIXING APPARATUS**

(30) Priority: 26.09.2022 WO PCT/CN2022/121280
(71) Applicant: Medtrum Technologies Inc., Shanghai 201203 (CN)
(72) Inventor: YANG, Cuijun, Shanghai 201203 (CN)
(74) Representative: Vogelbruch, Keang
(86) International application number: PCT/CN2023/092239
(87) International publication number: WO 2024/066357

(57) **Abstract**

The invention discloses an installation unit for an analyte sensor, the buckle of the parallel slider module is engaged with the annular structure of the analyte detecting device, so that a releasable connection is formed between the parallel slider module and the analyte detecting device, the analyte detecting device is fixed on the parallel slider module when the parallel slider module is located at the distal end, and the analyte detecting device is automatically separated from the parallel slider module when the parallel slider module slides to the proximal end, so that the installation unit is simple, reliable and easy to use.

## Description

### TECHNICAL FIELD

The invention mainly relates to the field of medical devices, in particular to an installation unit for an analyte detection device.

### BACKGROUND

The pancreas in the healthy human body can automatically monitor the glucose level and automatically secrete required amount of insulin/glucagon. For the type 1 diabetes patient, the pancreas does not function properly and cannot secrete enough insulin for the body. Therefore, type 1 diabetes is the metabolic disease caused by abnormal pancreatic function, and diabetes is a chronic disease. At present, there is no cure for diabetes with medical technology. The onset and development of diabetes and its complications can only be controlled by stabilizing glucose.

Diabetics need to have their glucose measured before they inject insulin into the body. At present, most of the testing methods can continuously measure glucose level and transmit the data to the remote device in real-time for the user to view. This method is called Continuous Glucose Monitoring (CGM). The method requires the detection device to be attached to the surface of the skin and the sensor it carries to be pierced into the tissue to complete the detection.

Therefore, the prior art urgently needs a simple and easy-to-use installation unit for the analyte detection device.

### BRIEF SUMMARY OF THE INVENTION

The invention discloses an installation unit for an analyte sensor, the buckle of the parallel slider module is engaged with the annular structure of the analyte detecting device, so that a releasable connection is formed between the parallel slider module and the analyte detecting device, the analyte detecting device is fixed on the parallel slider module when the parallel slider module is located at the distal end, and the analyte detecting device is automatically separated from the parallel slider module when the parallel slider module slides to the proximal end, so that the installation unit is simple, reliable and easy to use.

The invention provides an installation unit for an analyte sensor, which at least comprises a housing, an auxiliary-needle and a parallel slider module, wherein in use of the installation unit, the auxiliary-needle and the parallel slider module are slid from a distal end to a proximal end within the housing, and at the proximal end, the analyte detection device is mounted on a surface of a user's skin for obtaining in vivo analyte parameter information; when the parallel slider module located at the distal end, a buckle of the parallel slider module is engaged with an annular structure of the analyte detecting device so that the analyte detecting device is secured to the parallel slider module, and wherein after the parallel slider module is slid to the proximal end, the buckle is disengaged with the annular structure and the analyte detecting device is separated from the parallel slider module.

According to one aspect of the invention, a shell of the analyte detection device comprises an upper-outer-shell and a lower-outer-shell.

According to one aspect of the invention, the annular structure surrounds an outside of the upper-outer-shell and/or the lower-outer-shell.

According to one aspect of the invention, the annular structure surrounds a joint of the upper-outer-shell and the lower-outer-shell.

According to one aspect of the invention, the upper-outer-shell and the lower-outer-shell have different circumferential dimensions.

According to one aspect of the invention, the circumferential dimension of the upper-outer-shell is larger than the circumferential dimension of the lower-outer-shell, the buckle engaged with the upper-outer-shell.

According to one aspect of the invention, the circumferential dimension of the upper-outer-shell is 0.05-5mm larger than the circumferential dimension of the lower-outer-shell.

According to one aspect of the invention, further comprises an inclined transition surface connecting outer edges of the upper-outer-shell and the lower-outer-shell, the buckle engaged with the inclined transition surface.

According to one aspect of the invention, the annular structure is an annular groove and the buckle engaged with the annular groove.

According to one aspect of the invention, the buckle is a T-shaped-structure buckle.

According to one aspect of the invention, the number of T-shaped-structure buckle is 3, evenly spaced on the parallel slider module.

According to one aspect of the invention, further comprises at least 1 set of matched limit block and limit hole.

According to one aspect of the invention, wherein the limit block is located on an inner circumference of the parallel slide module and the limit hole is located on an outer circumference of the analyte detection device.

According to one aspect of the invention, wherein the limit block is located on an outer circumference of the analyte detection device and the limit hole is located on an inner circumference of the parallel slide module.

According to one aspect of the invention, further comprises at least one through-hole located on the shell.

According to one aspect of the invention, the at least one through-hole includes a first through-hole located in the lower-outer-shell and a second through-hole located in the upper-outer-shell, the first through-hole and the second through-hole is coaxial.

According to an aspect of the invention, the auxiliary-needle passes through the first through-hole and the second through-hole when the limit block is embedded in the limit hole.

According to one aspect of the invention, further comprises a self-sealing member disposed on the shell.

According to one aspect of the invention, the auxiliary-needle passes through the self-sealing member when the limit block is embedded in the limit hole.

According to one aspect of the invention, the analyte detection device further comprises a sensor, a transmitter, an internal circuitry and a battery, the sensor comprising an internal part and an external part, the external part, the transmitter, the internal circuitry and the battery are disposed within the shell.

According to one aspect of the invention, the internal part is bent relative to the external part.

According to one aspect of the invention, the external part is electrically coupled to the internal circuitry.

According to one aspect of the invention, further comprises a conductive strip, the external part is electrically coupled to the internal circuitry via the conductive strip.

According to one aspect of the invention, the conductive strip consists of spaced conductive zones and insulating zones.

Compared with the prior art, the technical scheme of the invention has the following advantages:
In installation unit for the analyte detection device disclosed by the invention, the buckle of the parallel slider module engages with the annular structure of the analyte detection device to realize the releasable connection between the parallel slider module and the analyte detection device, which make the installation unit simple, easy to use, and reliable.

Further, the annular structure may be an annular groove surrounding the outer edge of the upper-outer-shell or the outer edge of the lower-outer-shell, which is simple and easy to process.

Further, the annular structure may be a step structure formed by the difference in circumferential dimensions of the upper-outer-shell and the-lower-outer shell, and the stepped structure can be formed without additional processing to the upper-outer-shell or the lower-outer-shell, which is simple and easy to process.

Further, the step structure may be a step structure plunging from the upper-outer-shell to the lower-outer-shell, which is simple and easy to process.

Further, the step structure may be an inclined transition surface connected from the outer edge of the upper-outer-shell to the outer edge of the lower-outer-shell, which can prevent the upper-outer-shell and lower-outer-shell from detaching because the user touched the outer edges of the upper-outer-shell and lower-outer-shell during use.

Further, at least a set of matched a limit block and a limit hole is also included, the limit block and the limit hole being disposed on the parallel slider module or the analyte detecting device, respectively, so that when the analyte detecting device is needed to be fixed on the parallel slider module, the direction of placing the analyte detecting device on the parallel slider module can be guided, so as to enable the auxiliary-needle to pass through the through hole or the self-sealing member on the analyte detecting device, so that the sensor can be enveloped within the auxiliary-needle, and at the same time, preventing the analyte detection device from rotationally deflecting on the parallel slider module.

### BRIEF DESCRIPTION OF THE DRAWINGS

Fig. 1 is a schematic diagram of the external structure of the installation unit of the analyte detection device according to an embodiment of the invention;
Fig. 2a is a schematic diagram of external structure of the housing according to an embodiment of the invention;
Fig. 2b is a schematic diagram of a protective cover according to an embodiment of the invention;
Fig. 3 is a schematic diagram of the explosive structure of the installation unit of the analyte detection device according to an embodiment of the invention;
Fig. 4 is a schematic diagram of the internal structure of the housing according to an embodiment of the invention;
Fig. 5a is a structural schematic diagram of the far end of the parallel slider module according to an embodiment of the invention;
Fig. 5b is a structural schematic diagram of the near end of the parallel slider module according to an embodiment of the invention;
Fig. 5c is a structural schematic diagram of the parallel slider module set with a limit block according to an embodiment of the invention;
Fig. 6a is a schematic diagram of the analyte detection device according to an embodiment of the invention;
Fig. 6b is another schematic structural diagram of the analyte detecting device according to an embodiment of the invention;
Fig. 6c is a schematic structural diagram of the analyte detecting device secured to a parallel slider module according to an embodiment of the invention;
Fig. 6d is the schematic structural diagram of the section A-A' of Fig. 6a according to an embodiment of the invention;
Fig. 6e-Fig. 6h are schematic structural diagrams of the conductive strip in different using methods according to an embodiment of the invention;
Fig. 7a is the structural diagram of the auxiliary-needle module according to an embodiment of the invention;
Fig. 7b is the bending diagram of the sensor according to an embodiment of the invention;
Fig. 7c is the schematic diagram showing that the elastic washer is arranged outside the auxiliary-needle before installation according to an embodiment of the invention;
Fig. 7d is the schematic diagram of the elastic washer set outside the auxiliary-needle after the auxiliary-needle is inserted into the subcutaneous according to an embodiment of the invention;
Fig. 7e is the schematic diagram showing that the elastic washer is arranged outside the sensor after the auxiliary-needle is retracted according to an embodiment of the invention;
Fig. 7f is the structural diagram of solid elastic washer according to an embodiment of the invention;
Fig. 7g is the structural diagram of hollow elastic washer according to an embodiment of the invention;
Fig. 7h is the structural diagram of the elastic washer embedded in the shell of the analyte detection device according to an embodiment of the invention;
Fig. 8 is the schematic diagram of the triggering module according to an embodiment of the invention;
Fig. 9 is the top view of the installation unit according to an embodiment of the invention;
Fig. 10a is the schematic diagram of section A structure in Fig. 9;
Fig. 10b is the schematic diagram of section B structure in Fig. 9;
Fig. 10c is the schematic diagram of section C structure in Fig. 9;
Fig. 11 is the bending schematic diagram of the first buckle because of force according to an embodiment of the invention.

### DETAILED DESCRIPTION

As mentioned above, the installation unit for the analyte detection device in the prior art is complex in structure and installation process, which result in higher production costs and inconvenience to the user.

In order to solve this problem, the invention provides an installation unit for an analyte sensor, the buckle of the parallel slider module is engaged with the annular structure of the analyte detecting device, so that a releasable connection is formed between the parallel slider module and the analyte detecting device, the analyte detecting device is fixed on the parallel slider module when the parallel slider module is located at the distal end, and the analyte detecting device is automatically separated from the parallel slider module when the parallel slider module slides to the proximal end, so that the installation unit is simple, reliable and easy to use.

Various exemplary embodiments of the invention will now be described in detail with reference to the attached drawings. It is understood that, unless otherwise specified, the relative arrangement of parts and steps, numerical expressions and values described in these embodiments shall not be construed as limitations on the scope of the invention.

In addition, it should be understood that the dimensions of the various components shown in the attached drawings are not necessarily drawn to actual proportions for ease of description, e. g. the thickness, width, length or distance of some elements may be enlarged relative to other structures.

The following descriptions of exemplary embodiments are illustrative only and do not in any sense limit the invention, its application or use. Techniques, methods and devices known to ordinary technicians in the relevant field may not be discussed in detail here, but to the extent applicable, they shall be considered as part of this manual.

It should be noted that similar labels and letters indicate similar items in the appending drawings below, so that once the item is defined or described in one of the appending drawings, there is no need to discuss it further in the subsequent appending drawings.

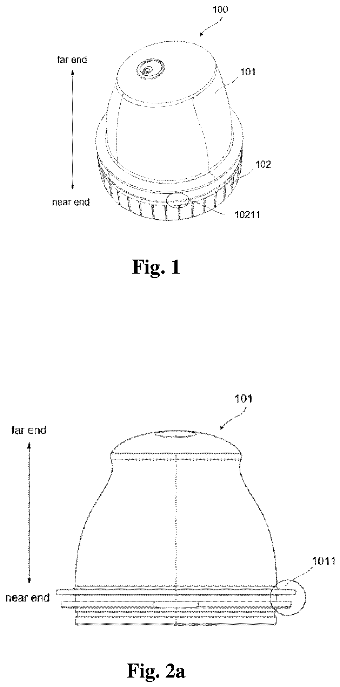

Fig. 1 is a schematic diagram of the external structure of the installation unit of the analyte detection device in an embodiment of the invention. The external structure of the installation unit 100 comprises a housing 101 for bearing the internal structural elements and a protective cover 102. The end of the installation unit 100 near the user's skin is the near end, and the end away from the skin is the far end. A first opening is arranged in the near end of housing 101. The protective cover 102 is used to protect, seal and prevent triggering of the internal structure and internal structural parts of the housing 101.

### External structure of the housing

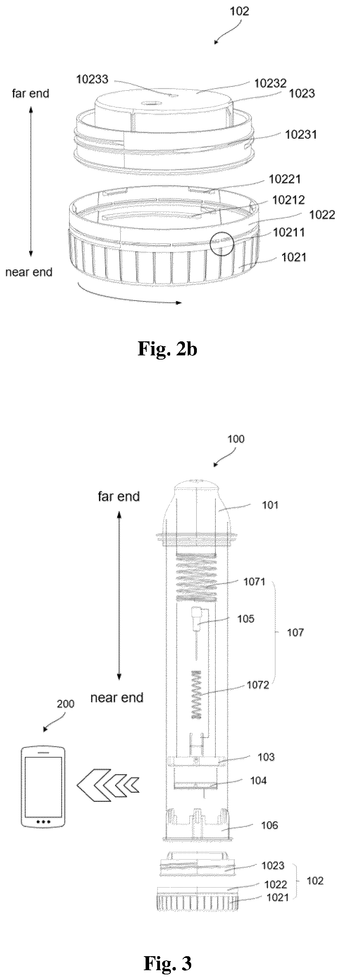

Fig. 2a is a schematic diagram of the external structure of the housing in an embodiment of the invention, and Fig. 2b is a schematic diagram of the protective cover. Protective cover 102 comprises outer cover 1021, clamp 1022 and inner cover 1023. The outer cover 1021 is provided with a second opening in the far end which faces the first opening. At the second opening, the outer cover 1021 is connected to the clamp 1022 by a breakable column 10211, which is distributed between the outer cover 1021 and the clamp 1022 at regular intervals. Column 10211 can be broken when the outer cover 1021 rotates relative to the clamp 1022, and the outer cover 1021 is separated from the clamp 1022.

The inner side of the outer cover 1021 is provided with an internal thread 10212, and the outer side of the inner cover 1023 is provided with an external thread 10231. The internal thread 10212 and the external thread 10231 can be connected to connect the outer cover 1021 and the inner cover 1023 together and keep fixed.

The inner side of the clamp 1022 is provided with a bulge 10221. Correspondingly, the outer side of the housing 101 is provided with a groove 1011, which forms a circle around the outer side of the housing and the bulge 10221 can be embedded into the groove 1011. The outer cover 1021 is fixed with the inner cover 1023 by screw thread, and then connected with the housing 101 by clamp 1022. The outer cover 1021 and the inner cover 1023 can protect, seal and prevent triggering of the internal structure of the housing 101. The anti-triggering function will be further described in the following.

In other embodiments of the invention, the outer cover 1021 and the inner cover 1023 can also be fixed by friction matching or buckle matching.

In other embodiments of the invention, the connection between clamp 1022 and housing 101 can also be realized by friction, buckle or thread.

### Internal structure of the housing

Fig. 3 is the schematic diagram of explosive structure of the installation unit of analyte detection device in embodiment of the invention, and the dotted line in the Fig. represents the installation and coordination relationship of each structural component. The internal structure of installation unit 100 of the analyte detection device comprises parallel slider module 103, analyte detection device 104, auxiliary-needle module 105, triggering module 106 and elastic module 107, which comprises the first elastic component 1071 and the second elastic component 1072.

The analyte detection device 104 and the installation unit 100 are not limited to the mating relationship described in Fig. 3. In some embodiments of the present invention, before installation, the analyte detection device 104 is built into the installation unit 100, and the user, when using, removes the installation unit 100 from the box to install the analyte detection device 104 onto the surface of human skin. In some other embodiments of the present invention, before installation, the analyte detection device 104 and the installation unit 100 are in a separate state, i.e., the user needs to take out the installation unit 100 and the analyte detection device 104 from the box separately and then the analyte detection device 104 can be installed onto the surface of the human body skin after the analyte detection device 104 is assembled into the installation unit 100.

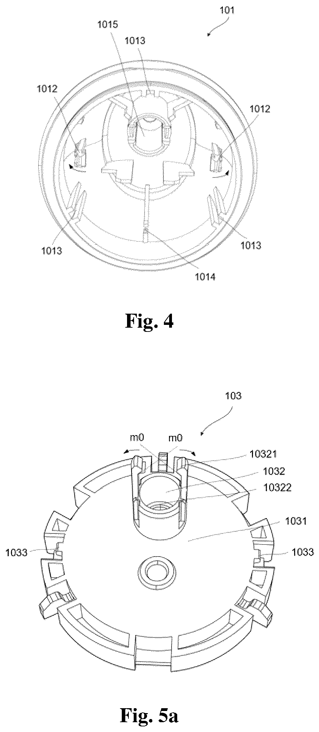

Fig. 4 is a schematic diagram of the internal structure of housing 101 in embodiment of the invention.

In an embodiment of the invention, at least two first buckles 1012 are arranged in the housing 101. The first buckle 1012 is integrated with the housing 101 and protrudes toward the near end of the housing 101. The first buckle 1012 is of flexible material and its end can be bent towards the outside of the housing 101.

In the preferred embodiment of the invention, the number of the first buckle 1012 is two, symmetrically distributed inside the housing 101, and the angle interval between each other is 180°.

In other preferred embodiments of the invention, the number of the first buckle 1012 is three or four, symmetrically distributed inside the housing 101, and the angle interval between them is 120° or 90°. The number of the first buckle 1012 can also be five or more, there is no limit here.

In the embodiment of the invention, housing 101 is also provided with at least two limit slots 1013, at least two slots 1014 and an auxiliary-needle limit slot 1015.

In an embodiment of the invention, the limit slot 1013 comprises at least two ribbed plates projecting from the inner wall of housing 101. In preferred embodiments of the invention, the ribbed plates are parallel to each other and grooves are formed in the middle of adjacent ribbed plates.

In other embodiments of the invention, the limit slot 1013 is a slot that is sunken into the inner wall of housing 101.

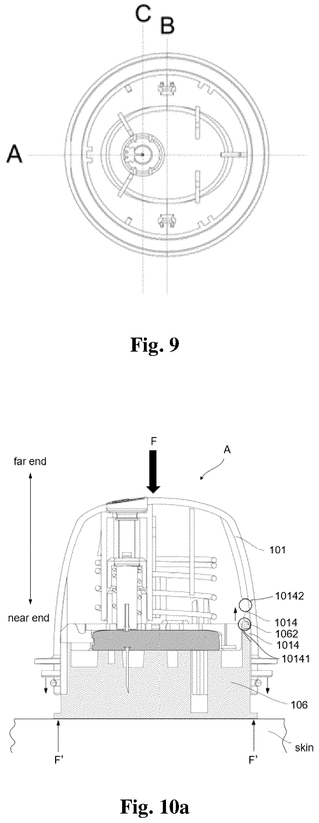

In the embodiment of the invention, slot 1014 comprises two slots, namely, the first slot point 10141 and the second slot point 10142, as shown in Fig. 10a, the first slot point 10141 is closer to the near end relative to the second slot point 10142.

In the preferred embodiment of the invention, there are two limit slots 1013 and slots 1014, which are symmetrically distributed inside the housing 101 with an angle interval of 180° between them.

In other preferred embodiments of the invention, there are three or four limit slots 1013 and slots 1014, which are symmetrically distributed inside housing 101 with an angle interval of 120° or 90° between them. The number of limit slots 1013 and slots 1014 can also be five or more, there is no limit here.

### Parallel slider module

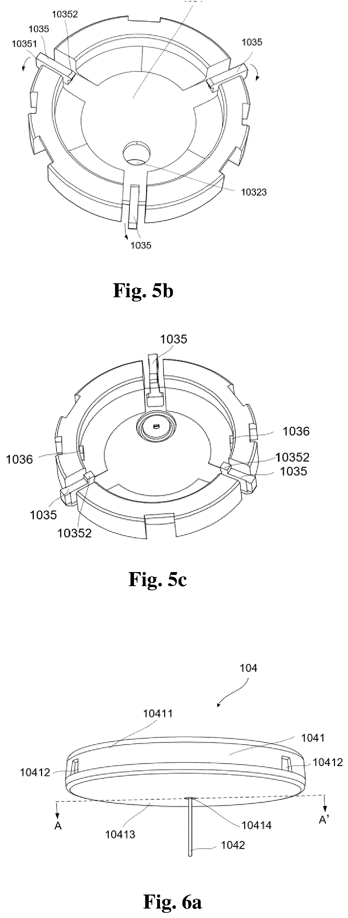

Fig. 5a is the structural diagram of the far end plane of parallel slider module 103, and Fig. 5b is the structural diagram of the near end plane of parallel slider module 103, Fig. 5c is a structural schematic diagram of the parallel slider module 103 set with a limit block 1036.

In the embodiment of the invention, the far end plane 1031 of the parallel slider module 103 is provided with a circular groove 1032 that protrudes to the far end. The circular groove 1032 is an internal hollowed cylindrical structure, and its inner diameter is d1. At least two slide-buckle 10321 are extended from the side wall of circular groove 1032 to the far end. The buckle part of the slide-buckle 10321 is plane or nearly plane, and is at a fixed angle with the horizontal plane, and its extended end m0 converges at the far end.

In an embodiment of the invention, the slide-buckle 10321 is a flexible material and can therefore be bent to the outside of the circular groove 1032.

In other embodiments of the invention, the slide-buckle 10321 may be directly arranged on the far end plane of the parallel slider module 103 without the need for a circular groove structure.

In the embodiment of the invention, the end of the circular groove 1032 near the far end plane 1031 is also provided with a boss 10322. The boss 10322 is an internally hollowed-out cylindrical structure with an inner diameter of d2, which can be understood as d1 > d2. The circular groove 1032 and the boss 10322 of the internal hollowed-out structure form a through-hole 10323 that runs from the far end plane 1031 to the near end plane 1034 of the parallel slider module.

In the preferred embodiment of the invention, the number of slide-buckle 10321 is two, symmetrically distributed on the side wall of circular groove 1032, and the angle interval between the two slide-buckle 10321 is 180°.

In other preferred embodiments of the invention, the number of slide-buckles 10321 can be three or four, symmetrically distributed on the side wall of circular groove 1032, and the angle interval between slide-buckles 10321 is 120° or 90°. The number of slide-buckles 10321 can also be five or more, there is no limit here.

According to Fig. 5a, in an embodiment of the invention, at least two second buckles 1033 are arranged on the side of the far end plane 1031 of parallel slider module 103. The second buckles 1033 are symmetrically distributed on the side of 1031 on the far end plane with an Angle interval of 180° between them.

In other embodiments of the invention, the number of the second buckles 1033 is three or four, symmetrically distributed on the side of the far end plane 1031, and the angle interval between them is 120° or 90°. The number of the second buckles 1033 can also be five or more, there is no limit here. In installation unit 100, the second buckle 1033 is coupled to the first buckle 1012. The position and number of the second buckle 1033 is the same as the first buckle 1012.

According to Fig. 5b, in the embodiment of the invention, at least two T-shaped structure 1035 are arranged on the side of the near end plane 1034 of parallel slider module 103. The vertical part of T-shaped structure 1035 is connected to the near end plane 1034, and the horizontal part comprises T-shaped-structure slider 10351 and T-shaped-structure buckle 10352. The T-shaped-structure slider 10351 is oriented to the outside of the parallel slider module 103 and protrudes out of the outer ring of the parallel slider module 103; T-shaped-structure buckle 10352 faces the inside of the parallel slider module 103 and protrudes from the inner ring of the parallel slider module 103.

In installation unit 100, T-shaped-structure slider 10351 is located in limit slot 1013 to limit the position of parallel slider module 103 and prevent rotation of parallel slider module 103. The number and position of T-shaped-structure slider 10351 are the same as that of limit slot 1013. In the process of moving the parallel slider module 103 towards the near end, the T-shaped-structure slider 10351 slides in the limit slot 1013.

In the preferred embodiment of the invention, the vertical part of T-shaped structure 1035 is a flexible material, the vertical part and the horizontal part are formed in one piece, and thus the horizontal part can be bent around the vertical part.

In other preferred embodiment of the invention, the vertical part of T-shaped structure 1035 is elastic material, such as spring, shrapnel, etc., and the horizontal part is fixedly connected with the vertical part through welding or hot melting processes, and the horizontal part can also be bent around the vertical part.

Referring to Fig. 5c, in other embodiments of the present invention, a limit block 1036 is also provided on the inner rim of the parallel slider module 103, which is used to limit the analyte detecting device 104 as well as to prevent rotational deflection of the analyte detecting device 104, as will be described below.

### Analyte detection device

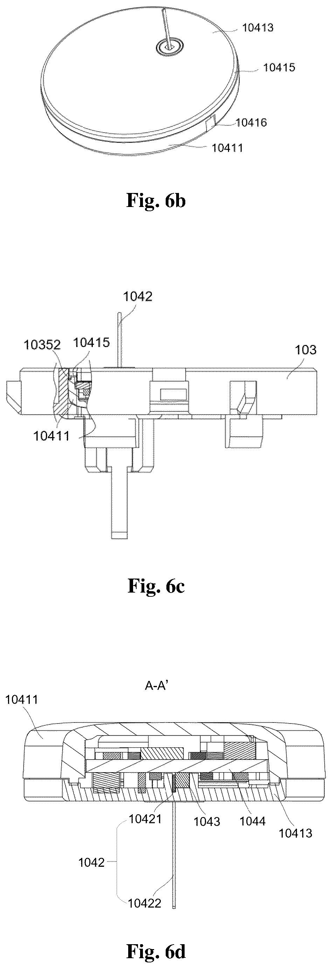

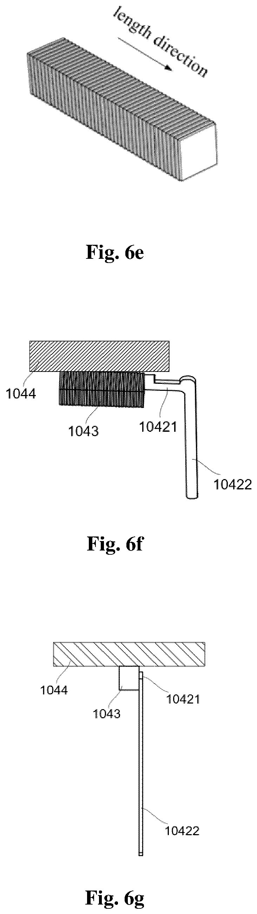

Fig. 6a is a schematic diagram of the analyte detection device in an embodiment of the invention; Fig. 6b is another schematic structural diagram of the analyte detecting device 104 of the embodiment of the present invention; Fig. 6c is a schematic structural diagram of the analyte detecting device 104 secured to a parallel slider module 103 of the embodiment of the present invention; Fig. 6d is the schematic structural diagram of the section A-A' of Fig. 6a; Fig. 6e-Fig. 6h are schematic diagrams of the conductive strip 1043 in different using methods.

In combination with Fig. 3, in an embodiment of the invention, the analyte detection device 104 comprises a shell 1041, a transmitter (not shown in the figure), a sensor 1042 and an internal circuit (not shown in the figure) arranged in the shell 1041 which electrically couples with the sensor. Sensor 1042 is used to detect the parameter information of user's body fluid analyte, and transmits the parameter information of the analyte to the transmitter through the internal circuit, and then from the transmitter to the external equipment 200.

In the preferred embodiment of the invention, before the analyte detection device 104 is installed on the user's skin surface, the signal is transmitted to the external device 200 at the first frequency f1, and after it is installed on the user's skin surface, the signal is transmitted to the external device 200 at the second frequency f2, and the second frequency f2 is greater than the first frequency f1. In further preferred embodiments of the invention, the first frequency f1 is 0~12 times/hour and the second frequency f2 is 12~3600 times/hour.

In the preferred embodiment of the invention, the first frequency f1 is 0 times/hour, that is, before the analyte detection device 104 is installed on the user's skin surface, no signal is transmitted to the external device 200, so as to save the power consumption of the analyte detection device 104 before installation.

In an embodiment of the invention, the shell 1041 comprises an upper-outer-shell 10411 and a lower-outer-shell 10413, and the upper-outer-shell 10411 and the lower-outer-shell 10413 are spliced together to form an internal space. The sensor 1042 consists of an external part (not shown) and an internal part (not shown). The external part, the transmitter and the internal circuit are arranged in the internal space and the external part is electrically coupled to the internal circuit. The internal part is provided with electrodes, membranes and other structures, which can be inserted into the user's skin to detect the parameters of the analyte. When the internal part is inserted under the skin, it needs to be inserted at the correct angle, such as perpendicular to the skin surface. At the end of its life, the analyte detection device 104 is removed from the user's skin surface and discarded as a whole.

In the embodiment of the invention, the lower-outer-shell 10413 comprises the first through-hole 10414. Correspondingly, on the axis of the first through-hole 10414, the upper-outer-shell 10411 comprises the second through-hole (not shown in the figure), and the internal part passes through the first through-hole 10414 to the outside of the shell, so as to insert the user's subcutaneous skin.

In other embodiments of the present invention, on the axis of the internal part, the upper-outer-shell 10411 is provided with a self-sealing member, which is initially in a sealed state, possesses a certain degree of elasticity and softness, and can be punctured by the auxiliary-needle 1052, such as a silicone, a butyl rubber, and the like. In the installation unit 100, the auxiliary-needle 1052 pierces the self-sealing member, and the self-sealing member will closely enclose the side wall of the auxiliary-needle 1052 without leaving gaps or openings, and after using the installation unit 100, the auxiliary-needle 1052 will be returned, and after the auxiliary-needle 1052 separated from the analyte detecting device 104, the self-sealing member will be recovered to the sealed state without leaving openings or holes, thus forming a good sealing on the analyte detecting device 104.

In other embodiments of the present invention, a self-sealing member is provided around the internal part of the lower-outer-shell 10413, the internal part passes through the self-sealing member, the self-sealing member surrounds the internal part, and the self-sealing member may provide sealing protection for the lower-outer-shell 10413 to prevent blood from being ejected or immersed into the analyte detecting device 104 when the internal part is pierced under the skin, and to provide waterproofing as well.

In other embodiments of the present invention, self-sealing members may be provided in both the upper-outer-shell 10411 and the lower-outer-shell 10413, or the self-sealing member may be provided in one of them. When the self-sealing member is provided in one of them, the other outer shell is provided with a through-hole for the auxiliary-needle 1052 to pass through.

In the embodiment of the invention, the self-sealing member disposed on the lower-outer-shell 10413 may be made of impermeable material that prevents outside light from reaching the interior of the analyte detection device 104 through the self-sealing member and triggering the photosensitive element.

In the embodiment of the present invention, the self-sealing member disposed on the upper-outer-shell 10411 may be made of light-transmissive material, and after the analyte detection device 104 has been ejected, outside light may pass through the self-sealing member to reach the interior of the analyte detection device 104 in order to trigger the photosensitive element.

In the embodiment of the invention, the side of the upper-outer-shell 10411 comprises buckle hole 10412 corresponding to T-shaped-structure buckle 10352, where "corresponding" means that the position and number of buckle hole 10412 are consistent with that of T-shaped-structure buckle 10352. In the installation unit 100, the upper-outer-shell 10411 is fitted with the near end plane 1034, the T-shaped-structure buckle 10352 forms a buckle-connection with the buckle hole 10412, and the analyte detection device 104 is fixed on the parallel slider module 103. When the horizontal part of the T-shaped structure 1035 is bent around the vertical part, the buckle-connection between the T-shaped-structure buckle 10352 and the buckle hole 10412 is removed, and the analyte detection device 104 is separated from the parallel slider module 103. Therefore, in the installation unit 100, the analyte detection device 104 and the parallel slider module 103 are in releasable connection.

In the embodiment of the present invention, the buckle hole 10412 corresponding to the T-shaped-structure buckle 10352, which not only make the analyte detection device 104 to be fixed to the parallel slider module 103, but also can be acted as a locator. The outer shell of the analyte detection device 104 is round, so when the analyte detection device 104 is mounted on the parallel slider module 103 before shipment, it is not possible to directly align the first through-hole 10414 with the auxiliary-needle 1052, and even after the first through-hole 10414 is aligned with the auxiliary-needle 1052 during mounting, during subsequent transportation and use, the analyte detection device 104 may still be rotational offset with respect to the parallel slide module 103, resulting in the internal part 1042 detaching from the envelope of the auxiliary-needle 1052 and affecting use. Therefore, according to the correspondence between the buckle holes 10412 and the T-shaped-structure buckle 10352, on the one hand, it is possible to determine the placement position of the analyte detecting device 104 on the parallel slider module 103, and on the other hand, it is possible to prevent the analyte detecting device from rotationally deflecting relative to the parallel slider module 103, which improves the use experience.

In the embodiment of the present invention, the circumferential dimensions of the upper-outer- shell 10411 and the lower-outer-shell 10413 are the same, so as to form a smooth connecting surface at the joint of the upper-outer-shell 10411 and the lower-outer-shell 10413, reducing the possibility of separation of the upper-outer-shell 10411 and the lower-outer-shell 10413 due to accidental touching, and ensuring the normal use of the analyte detection device 104.

In the embodiment of the present invention, an annular groove is provided around the upper-outer-shell 10411 on the outer side of the upper-outer-shell 10411, and the T-shaped-structure buckle 10352 can be embedded in the annular groove to realize snap fastening of the analyte detecting device 104. After the T-shaped-structure buckle 10352 is detached from the annular groove, the analyte detecting device 104 can be detached from the parallel slider module 103.

In other embodiments of the present invention, the annular groove may also be provided on the outer side of the lower-outer-shell 10413, or on the outer side of the joint between the upper-outer-shell 10411 and the lower-outer-shell 10413.

Referencing to Fig. 6b and Fig. 6c, in other embodiments of the present invention, the circumferential dimensions of the upper-outer-shell 10411 and the lower-outer-shell 10413 aren't the same, and preferably, the diameter of the upper-outer-shell 10411 is slightly larger than the diameter of the lower-outer-shell 10413, e.g., the diameter of the upper-outer-shell 10411 is 0.05 to 5 mm larger than the diameter of the lower-outer-shell 10413, such that the upper-outer-shell 10411 forms a step structure 10415 with respect to the lower-outer-shell 10413.When the analyte detection device 104 is placed on the parallel slider module 103, the upper outer shell 10411 fits against the near end plane 1034, the T-shaped-structure buckle 10352 fits against the step structure 10415 to form a snap connection, and the T-shaped-structure buckle 10352 presses the analyte detection device 104 against the lower outer shell 10413 through the step structure 10415.

In some embodiments of the present invention, the step structure 10415 may be a step structure plunging from the upper-outer-shell 10411 to the lower-outer-shell 10413 (as shown in Fig. 6a), and when the parallel slider module 103 is located at the distal end, the analyte detecting device 104 is fixed to the parallel slider module 103, and T-shaped-structure buckle 10352 engages the upper-outer-shell 10411.However, in the embodiment of the present invention, after the analyte detection device 104 is mounted to the surface of the user's skin, during using, the user may touch the outer edge of the step structure, causing the upper-outer-shell 10411 to detach from the lower-outer-shell 10413, which exposes the internal components of the analyte detection device 104 and affects the reliability of using the analyte detection device 104.

In response to the problems that may exist with the above step structure, in other embodiments of the present invention, the step structure 10415 may also include an inclined transition surface connected from the outer edge of the upper-outer-shell 10411 to the outer edge of the lower-outer -shell 10413, which may be a shell extension of the upper-outer-shell 10411 or a shell extension of the lower-outer-shell 10413, and the inclined transition surface may connect the outer edge of the upper-outer-shell 10411 and the outer edge of the lower-outer-shell 10413 closely together, so that when a user touches the outer edges of the upper-outer-shell 10411 or the lower-outer-shell 10413 during use, it will not result in the separation of the upper-outer-shell 10411 and the lower-outer-shell 10413, which improves the reliability of the use of the analyte detection device. When the parallel slider module is located at the distal end, the analyte detection device 104 is fixed to the parallel slider module 103, and the T-shaped-structure buckle 10352 engages the inclined transition surface.

It will be appreciated by those skilled in the art that whether the step structure 10415 is a step structure or an inclined transition surface, the step structure 10415 surrounds the outer side of the shell 1041 in an annular structure.

With reference to Fig. 5c, in the embodiment of the present invention, the analyte detection device 104 can be fixed on the parallel slide module 103 by the cooperation of the step structure 10415 and the T-shaped-structure buckle 10352, but it cannot be acted as a locator as previously described, i.e., it is not possible to prevent the analyte detection device 104 from rotational offset, based on which, it is also necessary to set up a limit hole 10416 on the shell 1041, correspondingly, a limit block 1036 is provided on the parallel slider module 103, and when the analyte detection device 104 is placed on the parallel slider module 103, the limit block 1036 can be embedded in the limit hole 10416, which on the one hand enables the analyte detection device 104 to be placed on the parallel slider module 103 according to a predetermined direction, and on the other hand, prevents the analyte detection device 104 from rotationally shifting, ensuring that the auxiliary-needle 1052 can pass through the first through-hole 10414 or the self-sealing member.

In the embodiment of the present invention, the number of limit blocks 1036 is at least 1. As may be known to the person skilled in the art, 1 limit block 1036 and 1 corresponding limit hole 10416 can realize the functions of positioning and preventing rotational offset, but it may lead to uneven force on the analyte detecting device 104, and therefore it is preferable to set at least 2 corresponding limit blocks 1036 and limit holes 10416, and the limit blocks 1036 and limit holes 10416 are distributed evenly on the circumference of the parallel slider module 103 and the analyte detecting device 104 respectively.

In the preferred embodiment of the present invention, the number of both the limit blocks 1036 and the limit holes 10416 are 2, and are symmetrically distributed on the circumferences of the parallel slider module 103 and the analyte detection device 104, respectively.

In the embodiment of the present invention, the limit blocks 1036 is provided on the inner circumference of the parallel slider module 103, and the limit holes 10416 are provided on the outer circumference of the shell of the analyte detection device 104.

Referring to Fig. 6d, Fig. 6d is a schematic view of the interior of the analyte detection device 104. As previously described, the analyte detection device 104 contains the internal part 10421 of the sensor 1042, the conductive strip 1042, and the internal circuitry 1044, and it will be appreciated by those of skill in the art that, in addition to the above-described components, the analyte detection device 104 may contain components such as a battery (not shown in the figures), a buzzer (not shown in the figures), and an LED light (not shown in the figures).

In the embodiment of the present invention, the internal part 10421 is provided with several pins, each of which is connected to the electrodes of the external part10422 by means of wires provided on an insulating substrate of the sensor 1042 for receiving a current from the internal circuitry 1044, and for transmitting a detection current containing information on analyte parameters to the internal circuitry 1044.

In some embodiments of the present invention, the internal part 10421 may directly contact the electrical contacts on the internal circuit 1044, but due to the size limitation, the pins on the internal part 10421 are densely distributed, and if it directly contacts the electrical contacts on the internal circuit 1044, it may result in some pins contacting the wrong electrical contacts, which may result in a short-circuit or a messy electrical signal.

Referring to Fig. 6e, in some embodiments of the present invention, the internal part 10421 is in contact with electrical contacts on the internal circuitry 1044 by means of a conductive strip 1043, which comprising spaced-apart conductive zones and insulating zones, the conductive zones and insulating distributed over the surface of the conductive strip 1043, or running through the conductive strip 1043.

In some embodiments of the present invention, in order to form a good conductive and insulating connection between the internal part 10421 and the internal circuitry 1044, and also to utilize as much as possible of the internal space of the analyte detecting device 104, some adjustments and improvements may be made to the relative positions and connection relationships of the sensor 1042, the conductive strip 1043, and the internal circuitry 1044.

In some embodiments of the present invention, the internal part 10421 of the sensor 1042 may be bent or curved with respect to the external part 10422. The bent or curved sensor 1042 is designed to utilize the interior space of the analyte detection device 104 better, and it will not compromise its use.

Continuing to refer to Fig. 6e, in some embodiments of the present invention, the electrically conductive strip 1043 is of rectangular type, with the electrically conductive and insulating zones spaced apart along its length, so that there are four electrically conductive contacting surfaces that can be used, and the four surfaces of the electrically conductive strip 1043 can be used flexibly according to the actual placement location and structural design of the sensor 1042 and the internal circuitry 1044.

In other embodiments of the present invention, the electrically conductive strip 1043 may also be of a regular type such as cylindrical, triangular, spherical, triangular-conical, or other irregular shapes.

Continuing to refer to Fig. 6d and the orientation shown in Fig. 6d. In this embodiment of the invention, the internal circuitry 1044 is placed inside the analyte detection device 104, and the conductive strip 1043 is of rectangular type, its upper surface in contact with the lower surface of the internal circuitry 1044, such that the conductive strip 1043 still has a left surface, a right surface, and a lower surface remaining to be accessible for contact with the internal part 10421 of the sensor 1042. In Fig. 6d, the external part 10421 is in contact with the left surface of the conductive strip 1043, and it is known to those skilled in the art that the external part 10421 can also be in contact with the lower surface or the right surface of the conductive strip 1043, as shown in Fig. 6g, which can also be effective in realizing the transfer of the current between the sensor 1042 and the internal circuitry 1044.

For simplicity in understanding the manner of electrical connection between the internal circuitry 1044, the conductive strip 1043, and the sensor 1042, the following descriptions are centered only on the three components and connections described above.

Referring to Fig. 6f, in some embodiments of the present invention, the external part 10421 is bent with respect to the internal part 10422, the external part 10421 is in contact with the right surface of the conductive strip 1043, and the upper surface of the conductive strip 1043 is in contact with the lower surface of the internal circuit 1044.

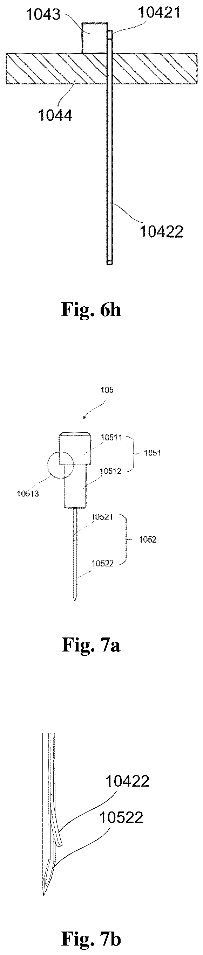

Referring to Fig. 6h, in some embodiments of the present invention, the lower surface of the conductive strip 1043 is in contact with the upper surface of the internal circuitry 1044, and the external part 10421 is in contact with the right surface of the conductive strip 1043. In some other embodiments of the present invention, the external part 10421 is bent or curved with respect to the internal part 10422, and in such a structure, the external part 10421 can be in contact with the upper surface of the electrically conductive strip 1043 without affecting the position and state of the internal part 10422.

In other embodiments of the present invention, the analyte detection device 104 is not limited to the above structure. In other embodiments of the present invention, the transmitter is provided outside the shell 1041, and the user mounts the transmitter to the shell 1041 after mounting the shell 1041 to the surface of the user's skin via the installation unit 100.

In further embodiments of the present invention, the battery is built into the transmitter when the transmitter is separated from the shell 1041.

In further embodiments of the present invention, the battery is built into the shell 1041 when the transmitter is separated from the shell 1041.

### Auxiliary-needle module

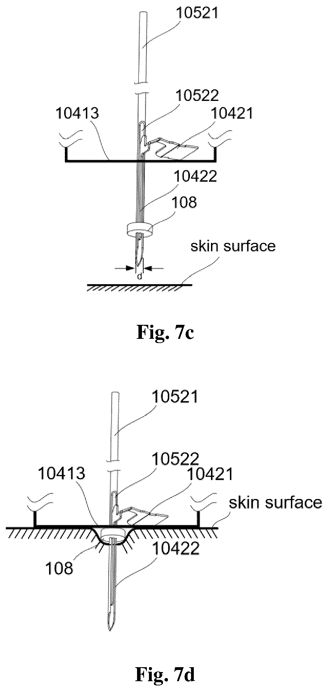

Fig. 7a is the structural diagram of the auxiliary-needle module in the embodiment of the invention. Fig. 7b shows the bending diagram of the sensor in the embodiment of the invention. Fig. 7c is a schematic diagram showing that the elastic washer is arranged outside the auxiliary-needle before installation in the embodiment of the invention. Fig. 7d is the schematic diagram of the elastic washer set outside the auxiliary-needle after the auxiliary-needle is inserted into the subcutaneous area in the embodiment of the invention.

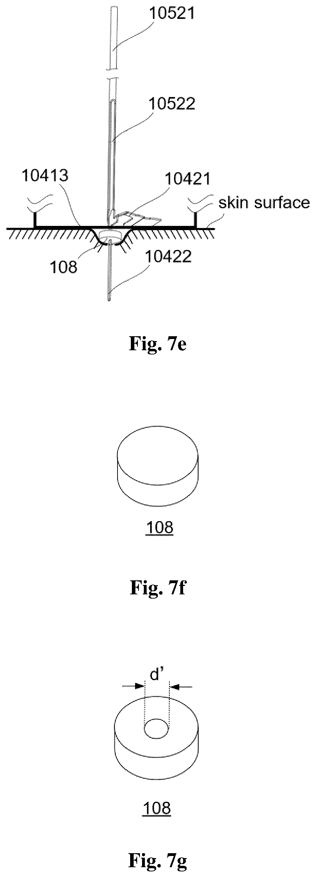

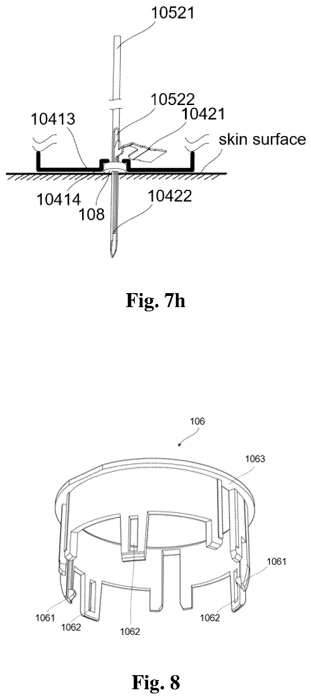

Fig. 7e is a schematic diagram of the elastic washer arranged outside the sensor after the auxiliary-needle is retracted in the embodiment of the invention. Fig. 7f is the structural diagram of the solid elastic washer in the embodiment of the invention. Fig. 7g is the structural diagram of the hollow elastic washer in the embodiment of the invention. Fig. 7h is the structural diagram of the elastic washer embedded in the shell of the analyte detection device in the embodiment of the invention.

Referring to Fig. 7a, in the embodiment of the invention, the auxiliary-needle module 105 comprises an auxiliary-needle fixed structure 1051 and an auxiliary-needle 1052. In the installation unit 100, the auxiliary-needle fixed structure 1051 is located at the far end, and the auxiliary-needle 1052 is located at the near end.

In the embodiment of the invention, the auxiliary-needle fixed structure 1051 comprises an auxiliary-needle slider 10511 and an auxiliary-needle fixed block 10512. The diameter or width of the auxiliary-needle slider 10511 is larger than that of the auxiliary-needle fixed block 10512, forming a convex face 10513 towards the near end.

In the embodiment of the invention, the auxiliary-needle 1052 comprises a fully-enclosed needle body 10521 and a semi-enclosed needle body 10522, which are located between the auxiliary-needle fixed block 10512 and the semi-enclosed needle body 10522, and are fixedly connected with the auxiliary-needle fixed block 10512. The hollow structure of the semi-enclosed needle body 10522 can be used to accommodate the internal part of the sensor 1042. When the semi-enclosed needle body 10522 is punctured under the skin of the user, the internal part can be punctured under the skin at the same time, and when the needle is retracted, the internal part will not be affected under the skin.

In other embodiments of the invention, the auxiliary-needle 1052 only comprises a semi-enclosed needle body 10522, that is, the semi-enclosed needle body 10522 is fixedly connected with the auxiliary-needle fixed block 10512, which can reduce the material consumption of the auxiliary-needle 1052 and save costs, but also reduce the rigidity of the auxiliary-needle 1052.

In the installation unit 100, the auxiliary-needle 1052 successively passes through the second through-hole and the first through-hole 10414, thus penetrating the analyte detection device 104. The internal part of the sensor 1042 is located in the semi enclosing needle body 10522.

With reference to Fig. 7b, although in an ideal state, it is expected that the half surrounding needle body 10522 can completely envelope the internal part 10422 of the sensor, since the internal part 10422 is arranged with electrodes (not shown in the figure) on the side close to the auxiliary-needle 1052, the base of the internal part 10422 will bend to the side far from the auxiliary-needle 1052, forming a state as shown in Fig. 7b. In addition, the size of each component in the installation unit 100 in the embodiment of the invention is small, which can reduce the weight and facilitate users, but during the installation process, due to the inconsistent size fit of each component, tolerance reservation and other reasons, such as the unstable fit between the limit slot 1013 and the T-shaped-structure slider 10351, the slider buckle 10321 and the auxiliary-needle slider 10511 buckle, the auxiliary-needle 1052 appears a slight swing, The semi-enclosed needle body 10522 and the internal part 10422 of the sensor cannot be in an effective parallel state, and the internal part 10422 may break away from the envelope of the semi-enclosed needle body 10522. Based on the above reasons, the internal part 10422 cannot be effectively inserted subcutaneously with the semi-enclosed needle body 10522, reducing the detection reliability of the analyte detection device 104.

Referring to Fig. 7c, in order to solve the above problem, in some embodiments of the invention, before installation, an elastic washer 108 is sleeved on the outside of the semi-enclosed needle body 10522, and the semi-enclosed needle body 10522 encloses the internal part 10422 of the sensor 1042. The elastic washer 108 can bind the internal part 10422 in the semi-enclosed needle body 10522, which can prevent the internal part 10422 from bending. At the same time, the auxiliary-needle 1052 can swing during installation, prevent the internal part 10422 from getting rid of the semi-enclosed needle body 10522, so that the internal part 1422 can smoothly insert the subcutaneous with the semi-enclosed needle body 10522, and improve the detection reliability of the analyte detection device 104.

In the preferred embodiment of the invention, the elastic washer 108 is close to the tip end of the semi-enclosed needle body 10522, which can better constrain the internal part 10422 and prevent the internal part 10422 from breaking away from the envelope of the semi-enclosed needle body 10522 or bending.

Referring to Fig. 7d, in some embodiments of the invention, when the installation unit 100 performs the installation action, the semi-enclosed needle body 10522 carries the internal part 10422 to insert the subcutaneous area, and the elastic washer 108 remains stationary due to the skin's obstruction. Therefore, during the process of penetrating the semi-enclosed needle body 10522 into the subcutaneous area, the elastic washer 108 slides to the far end relative to the semi-enclosed needle body 10522 until the lower-outer-shell 10413 of the analyte detection device 104 contacts the skin surface, and press the elastic washer 108.

Referring to Fig. 7e, in some embodiments of the invention, the semi-enclosed needle body 10522 carries the internal part 10422, which is punctured subcutaneously to a predetermined position and retracted, leaving the internal part 10422 under the skin to detect analyte parameter information. The analyte detection device 104 is stuck to the skin surface under the action of adhesive tape. Therefore, when the semi-enclosed needle body 10522 is retracted, the elastic washer 108 is blocked by the lower-outer-shell 10413 and remains stationary. The elastic washer 108 slides toward the near end relative to the semi-enclosed needle body 10522 until the semi-enclosed needle body 10522 is separated from the elastic washer 108. The elastic washer 108 is sandwiched between the lower-outer-shell 10413 and the skin surface. After the semi-enclosed needle body 10522 is fully retracted, the elastic washer 108, under the effect of its own elasticity, shrinks the sleeve to the outside of the internal part 10422, presses and covers the wound caused by the semi-enclosed needle body 10522 penetrating into the subcutaneous area, which can prevent blood from spilling from the wound and polluting the analyte detection device 104, and can also prevent foreign matters from polluting the wound and improve the wound healing speed.

Referring to Fig. 7f, in some embodiments of the invention, the elastic washer 108 can be a solid structure. After the semi-enclosed needle body 10522 pierces the elastic washer 108, the elastic washer 108 is sleeved outside the semi-enclosed needle body 10522.

With reference to Fig. 7c and Fig. 7g, in some embodiments of the invention, the elastic washer 108 can be hollow structure, and the inner diameter d' of the elastic washer 108 is less than the outer diameter d of the semi-enclosed needle body 10522, so that the elastic washer 108 can be closely sleeved on the outer side of the semi-enclosed needle body 10522 to prevent the internal part 10422 from separating from the envelope of the semi-enclosed needle body 10522. In the preferred embodiment of the invention, the inner diameter d' of the elastic washer 108 is still smaller than the outer diameter of the internal part 10422. After the semi-enclosed needle body 10522 is retracted, the elastic washer 108 can be shrunk and tightly sleeved on the outer side of the internal part 10422 to press and cover the wound caused by the semi-enclosed needle body 10522 penetrating into the subcutaneous.

In some embodiments of the invention, the thickness of the elastic washer 108 is 0.01-5mm. If the elastic washer 108 is too thick, the pressure on the skin surface of the analyte detection device 104 will increase, causing discomfort. If the elastic washer 108 is too thin, it is easy to deform itself, and cannot form an effective constraint on the internal part 10422. In the preferred embodiment of the invention, the thickness of the elastic washer 108 is 0.3mm.

Referring to Fig. 7h, in some embodiments of the invention, the first through-hole 10414 is internally sunk into the lower-outer-shell 10413. During installation, the elastic washer 108 is held against the skin surface until the lower-outer-shell 10413 is pushed into contact with the skin surface. The elastic washer 108 is embedded in the first through-hole 10414 and flush with the lower-outer-shell 10413, so that the elastic washer 108 will not cause additional extrusion on the skin surface, reducing discomfort.

In some embodiments of the invention, the material of the elastic washer 108 is one of rubber, silica gel or latex, which has good elasticity and can avoid polluting the wound.

### Triggering module

Fig. 8 is a schematic diagram of the triggering module in an embodiment of the invention.

In an embodiment of the invention, the triggering module 106 is provided with at least two fixed buckle 1061 corresponding to the first buckle 1012. In installation unit 100, the fixed buckle 1061 is in contact with the first buckle 1012 to prevent the first buckle 1012 from bending to the outside of the housing 101. The contact between the fixed buckle 1061 and the first buckle 1012 can be point-contact, line-contact or surface-contact. When the contact is surface-contact, the contact surface between the fixed buckle 1061 and the first buckle 1012 is at a fixed angle with the horizontal plane and converges at the near end of the installation unit 100. The number and position of the fixed buckle 1061 is the same as the first buckle 1012.

In the embodiment of the invention, there are also at least two lugs 1062 arranged on the triggering module 106. In the installation unit 100, the lug 1062 and the slot 1014 snap together to fix the triggering module 106. The number and position of lug 1062 are the same as slot 1014. According to Fig.10a, before installation unit 100 is used, lug 1062 is located in slot 10141 of the first buckle. At this point, the fixed buckle 1061 is in contact with the first buckle 1012.

In an embodiment of the invention, the triggering module 106 also comprises an outer ring 1063, which connects the fixed buckle 1061 and the lug 1062 into an integral whole. In installation unit 100, the outer ring 1063 is proximal to the first opening and protrudes from the first opening relative to the lug 1062. In using installation unit 100, the outer ring 1063 is attached to the user's skin surface.

### Elastic module

According to Fig. 3, the elastic module 107 comprises the first elastic component 1071 and the second elastic component 1072.

In the embodiment of the invention, the first elastic component 1071 is located between the parallel slider module 103 and the housing 101, that is, one end of the first elastic component 1071 is located on the far end plane of the parallel slider module 103, and the other end is located in the housing 101. In the installation unit 100, the first elastic component 1071 is in compression state and can provide elasticity.

In the embodiment of the invention, the second elastic component 1072 is located between parallel slider module 103 and auxiliary-needle module 105, that is, one end of the second elastic component 1072 is located on the boss 10322 of parallel slider module 103 and the other end is located on the convex surface 10513 of auxiliary-needle module 105. In the installation unit 100, the second elastic component 1072 is in a compressed state to provide elasticity.

In the preferred embodiment of the invention, the first elastic component 1071 or the second elastic component 1072 is a metal spring.

In the embodiment of the invention, the inner ring diameter of the first elastic component 1071 is larger than the outer ring diameter of the circular groove 1032 and the auxiliary-needle slide block 10511. In the installation unit 100, the first elastic component 1071 is surrounded by the outer side of the auxiliary-needle slide block 10511 and the circular groove 1032, so that the internal space of the installation unit 100 can be fully utilized.

In the embodiment of the invention, the outer ring diameter of the second elastic component 1072 is larger than the outer diameter of the auxiliary-needle fixed block 10512 and the inner diameter of the boss 10322, but smaller than the outer diameter of the auxiliary-needle slide block 10511 and the inner diameter of the circular groove 1032. Therefore, one end of the second elastic component 1072 is placed in the circular groove 1032. The other end is enclosed outside the auxiliary pin fixed block 10512 to make full use of the internal space of the installation unit 100.

### Use method of installation unit

Fig. 9 is the top view of the installation unit of the embodiment of the invention.

Fig. 10a is the schematic diagram of section A structure in Fig. 9.

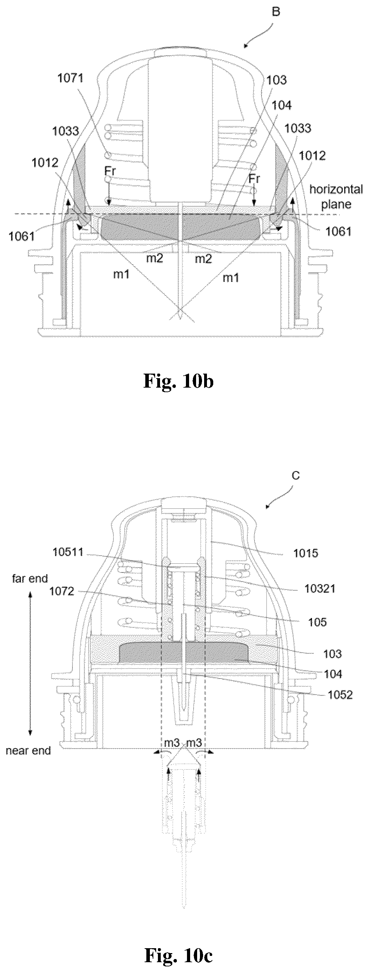

Fig. 10b is the schematic diagram of section B structure in Fig. 9.

Fig. 10c is the schematic diagram of section C structure in Fig. 9.

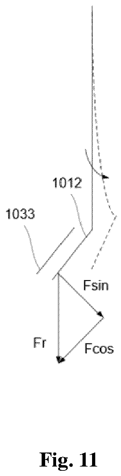

Fig. 11 shows the bending diagram of the first buckle.

According to Fig. 10a and Fig. 10b, in the embodiment of the invention, the slot 1014 is provided with the first slot point 10141 and the second slot point 10142. Before the installation unit 100 is used, triggering module 106 is fixed to the housing 101 through the coupling of lug 1062 and slot 10141, at this time, the fixed buckle 1061 is in contact with the first buckle 1012 to prevent the first buckle 1012 from bending to the outside of the housing 101. The fixed buckle 1061, the first buckle 1012 and the second buckle 1033 are at the same horizontal plane. In the preferred embodiment of the invention, the second buckle 1033, the first buckle 1012 and the fixed buckle 1061 are successively arranged from the housing 101 inside out.

In the embodiment of the invention, the contact between the fixed buckle 1061 and the first buckle 1012 is one of point-contact, line-contact or surface-contact. When the above contact is surface-contact, the extension line m1 of the contact surface converging at the near end. This kind of structural design can make the fixed buckle 1061 move toward the far end relative to the first buckle 1012.

In the preferred embodiment of the invention, the coupling surface of the second buckle 1033 and the first buckle 1012 is a plane, which has a fixed angle with the horizontal plane, and its extended line m2 converges at the near end.

With reference to Fig. 11, this kind of structural design can make the second buckle 1033 move toward the near end relative to the first buckle 1012, and push the first buckle 1012 toward the outside of the housing 101, so as to release the coupling state between the first buckle 1012 and the second buckle 1033.

In an embodiment of the invention, the first elastic component 1071 is in a state of compression and has elastic potential energy, and its self-elasticity gives thrust Fr to the near end of parallel slider module 103. The thrust Fr acts on the first buckle 1012 through the coupling surface of the second buckle 1033 and the first buckle 1012. And generate a component force Fsin perpendicular to the plane of the first buckle 1012, which can push the first buckle 1012 toward the outside of the housing 101 to bend, so as to release the coupling state of the first buckle 1012 and the second buckle 1033.

In the embodiment of the invention, when the installation unit 100 is used, the outer cover 1021 is rotated to break the column 10211, the protective cover 102 is separated from the housing 101, and the near end of the installation unit 100 is close to the user's skin until the outer ring 1063 of triggering module 106 is triggered to stick to the skin surface, and the user presses the housing 101 at the far end. The housing 101 moves toward the skin, while the triggering module 106 remains stationary. Therefore, the triggering module 106 moves toward the far end relative to the housing 101, and the lug 1062 disconnects from the first slot point 10141 and enters the second slot point 10142. Meanwhile, the fixed buckle 1061 no longer contacts with the first buckle 1012. The coupling state between the first buckle 1012 and the second buckle 1033 is released due to the bending of the component force Fsin toward the outside of the housing 101.

In the embodiment of the invention, after the coupling state is released, the parallel slider module 103 continues to move toward the near end under the elastic force of the first elastic component 1071, and drives the analyte detection device 104 to move toward the near end until the lower-outer-shell 10413 of the analyte detection device 104 contacts the user's skin surface.

According to Fig. 10c, in the embodiment of the invention, the slide-buckles 10321 is connected with the auxiliary-needle slide block 10511. When the first elastic component 1071 pushes the parallel slider module 103 to move toward the near end, the auxiliary-needle module 105 is driven to move toward the near end together.

In an embodiment of the invention, the connection point of slide-buckles 10321 and auxiliary-needle slide block 10511 is a plane or approximate plane, which has a fixed angle with the horizontal plane, and the extension line m3 converges at the far end. The thrust force of the second elastic component 1072 on the auxiliary-needle slide block 10511 is toward the far end, so the auxiliary-needle slide block 10511 can push the slide-buckles 10321 toward the outside of the housing 101, making the slide-buckles 10321 bend, the principle of which is equivalent to Fig. 11.

In the embodiment of the invention, in the installation unit 100, the side wall of the limit slot 1015 of the auxiliary-needle prevents the slide-buckles 10321 from bending, and the connection state of the slide-buckles 10321 and the auxiliary-needle slide block 10511 clasp does not change. As parallel slider module 103 and auxiliary-needle module 105 move towards the near end, the slide-buckles 10321 is separated from the auxiliary-needle limit slot 1015, and the inner wall of the auxiliary-needle limit slot 1015 no longer prevents the slide-buckles 10321 from bending. The second elastic component 1072 pushes the auxiliary-needle slide block 10511 to the far end. At the same time, the auxiliary-needle slide block 10511 pushes the slide-buckle 10321 to bend outwards, and the connection between the slide-buckle 10321 and the auxiliary-needle slide block 10511 is released. The second elastic component 1072 continues to push the auxiliary-needle slide block 10511 to move toward the far end, and finally the auxiliary-needle module 105 returns to its initial position. Auxiliary-needle 1052 retracted into housing 101 to prevent auxiliary-needle 1052 from being exposed to housing 101 and causing unnecessary damage.

In the embodiment of the invention, when the slide-buckles 10321 is detached from the limit slot 1015 of the auxiliary-needle, the semi-enclosed needle body 10522 of the auxiliary-needle inserts the user's subcutaneous.

In the embodiment of the invention, in the installation unit 100, the T-shaped-structure slider 10351 is located in the limit slot 1013, and the limit slot 1013 restricts the position and direction of the parallel slider module 103 through the T-shaped-structure slider 10351 to ensure that the parallel slider module 103 is perpendicular to its sliding direction. Thus, the analyte detection device 104 set at the front end of the parallel slider module 103 is kept perpendicular to its motion direction, and the auxiliary-needle 1052 is kept parallel to its motion direction, so that the part of the sensor inside the auxiliary-needle 1052 and its envelope can pierce the user's skin at a vertical angle to relieve the user's pain.

In the embodiment of the invention, in the process of parallel slider module 103 sliding towards the near end, the T-shaped-structure slider 10351 slides in the limit slot 1013 until it touches the outer ring 1063 of the triggering module 106. Driven by the first elastic component 1071, the parallel slider module 103 continues to move towards the near end. The outer ring 1063 stops the T-shaped-structure slider 10351 from moving toward the near end, so the T-shaped-structure slider 10351 is bent around the vertical part, the T-shaped-structure buckle 10352 is disconnected from the buckle hole 10412, and the analyte detection device 104 is separated from the parallel slider module 103, so that it can be installed on the user's skin surface.

In the embodiment of the invention, when the T-shaped-structure slider 10351 contacts with the outer ring 1063, the position of the parallel slider module 103 is a predetermined position. At this time, the lower-outer-shell 10413 of the analyte detection device contacts the user's skin surface.

In an embodiment of the invention, the auxiliary-needle 1052 passes through the second through-hole and the first through-hole 10414 in turn, and through the analyte detection device 104. Meanwhile, the semi-enclosed needle body 10522 of the auxiliary-needle 1052 envelope sensor 1042. In the process of parallel slider module 103 and auxiliary-needle module 105 moving towards the near end, the semi-enclosed needle body 10522 with sensor 1042 inserted into the subcutaneous body. After the auxiliary-needle 1052 retracted, the internal part of sensor 1042 remained under the subcutaneous body, and the state of the internal part of sensor 1042 was not affected when the auxiliary-needle 1052 retracted.

In the embodiment of the invention, the user is required to press the housing 101 at the far end and apply force F to the housing 101 at the near end to trigger the outer ring 1063 of triggering module 106 to contact the user's skin surface, and the user's skin provides force F 'of outer ring 1063 that is opposite to force F, so as to realize the relative movement of triggering module 106 and housing 101. During the actual installation, the absolute position of the triggering module 106 remains unchanged, and the housing 101 moves toward the near end.

Before the installation, in order to prevent the triggering module 106 from moving relative to the housing 101, a protective cover 102 is installed at the near end of the housing 101. The protective cover 102 is surrounded by the outer ring 1063 of the triggering module 106, so as to prevent the installation action from being carried out in an incorrect position due to the accidental collision of the outer ring 1063.

The distal face 10232 of the inner cover 1023 is in contact with the analyte detection device 104, a t the same time, the auxiliary-needle 1052 and the sensor 1042 are extended into the groove of the inner cover 10233, which can play a sealing role and prevent the outside dust, particles and other dirt from contacting the needle body and the sensor and causing pollution.

In embodiments of the invention, adhesive tape is also arranged on the lower-outer-shell 10413 (not shown in the figure) of the analyte detection device to fix the analyte detection device 104 on the user's skin surface.

To sum up, the embodiment of the invention discloses an installation unit for an analyte sensor, the buckle of the parallel slider module is engaged with the annular structure of the analyte detecting device, so that a releasable connection is formed between the parallel slider module and the analyte detecting device, the analyte detecting device is fixed on the parallel slider module when the parallel slider module is located at the distal end, and the analyte detecting device is automatically separated from the parallel slider module when the parallel slider module slides to the proximal end, so that the installation unit is simple, reliable and easy to use.

Although some specific embodiments of the invention have been detailed through examples, technicians in the field should understand that the above examples are for illustrative purposes only and are not intended to limit the scope of the invention. Persons skilled in the field should understand that the above embodiments may be modified without departing from the scope and spirit of the invention. The scope of the invention is limited by the attached claims.

## Claims

1. An installation unit for an analyte detection device, at least comprising:
a housing, an auxiliary-needle and a parallel slider module, wherein in use of the installation unit,
the auxiliary-needle and the parallel slider module are slid from a distal end to a proximal end within the housing and at the proximal end, the analyte detection device is mounted on a surface of a user's skin for obtaining in vivo analyte parameter information;
when the parallel slider module located at the distal end, a buckle of the parallel slider module is engaged with an annular structure of the analyte detecting device so that the analyte detecting device is secured to the parallel slider module, and wherein after the parallel slider module is slid to the proximal end, the buckle is disengaged with the annular structure and the analyte detecting device is separated from the parallel slider module.

2. The installation unit for the analyte detection device of claim 1, wherein a shell of the analyte detection device comprises an upper-outer-shell and a lower-outer-shell.

3. The installation unit for the analyte detection device of claim 2, wherein the annular structure surrounds an outside of the upper-outer-shell and/or the lower-outer-shell.

4. The installation unit for the analyte detection device of claim 3, wherein the annular structure surrounds a joint of the upper-outer-shell and the lower-outer-shell.

5. The installation unit for the analyte detection device of claim 2, wherein the upper-outer-shell and the lower-outer-shell have different circumferential dimensions.

6. The installation unit for the analyte detection device of claim 5, wherein the circumferential dimension of the upper-outer-shell is larger than the circumferential dimension of the lower-outer-shell, the buckle engaged with the upper-outer-shell.

7. The installation unit for the analyte detection device of claim 6, wherein the circumferential dimension of the upper-outer-shell is 0.05-5mm larger than the circumferential dimension of the lower-outer-shell.

8. The installation unit for the analyte detection device of claim 5, further comprises an inclined transition surface connecting outer edges of the upper-outer-shell and the lower-outer-shell, the buckle engaged with the inclined transition surface.

9. The installation unit for the analyte detection device of claim 3, wherein the annular structure is an annular groove and the buckle engaged with the annular groove.

10. The installation unit for the analyte detection device of any one of claim 1-9, wherein the buckle is a T- shaped-structure buckle.

11. The installation unit for the analyte detection device of claim 10, wherein the number of T-shaped-structure buckle is 3, evenly spaced on the parallel slider module.

12. The installation unit for the analyte detection device of claim 2, further comprises at least 1 set of matched limit block and limit hole.

13. The installation unit for the analyte detection device of claim 12, wherein the limit block is located on an inner circumference of the parallel slide module and the limit hole is located on an outer circumference of the analyte detection device.

14. The installation unit for the analyte detection device of claim 12, wherein the limit block is located on an outer circumference of the analyte detection device and the limit hole is located on an inner circumference of the parallel slide module.

15. The installation unit for the analyte detection device of claim 12, further comprises at least one through-hole located on the shell.

16. The installation unit for the analyte detection device of claim 15, wherein the at least one through-hole includes a first through-hole located in the lower-outer-shell and a second through-hole located in the upper-outer-shell, the first through-hole and the second through-hole is coaxial.

17. The installation unit for the analyte detection device of claim 16, wherein the auxiliary-needle passes through the first through-hole and the second through-hole when the limit block is embedded in the limit hole.

18. The installation unit for the analyte detection device of claim 12, further comprises a self-sealing member disposed on the shell.

19. The installation unit for the analyte detection device of claim 18, wherein the auxiliary-needle passes through the self-sealing member when the limit block is embedded in the limit hole.

20. The installation unit for the analyte detection device of claim 2, wherein the analyte detection device further comprises a sensor, a transmitter, an internal circuitry and a battery, the sensor comprising an internal part and an external part, the external part, the transmitter, the internal circuitry and the battery are disposed within the shell.

21. The installation unit for the analyte detection device of claim 20, wherein the internal part is bent relative to the external part.

22. The installation unit for the analyte detection device of claim 20, wherein the external part is electrically coupled to the internal circuitry.

23. The installation unit for the analyte detection device of claim 20, further comprises a conductive strip, the external part is electrically coupled to the internal circuitry via the conductive strip.

24. The installation unit for the analyte detection device of claim 20, wherein the conductive strip consists of spaced conductive zones and insulating zones.
